(19) Europäisches Patentamt
European Patent Office
Office européen des brevets

(11) Publication number : **0 450 706 B1**

(12) **EUROPEAN PATENT SPECIFICATION**

(45) Date of publication of patent specification : **08.06.94 Bulletin 94/23**

(51) Int. Cl.$^5$ : **C07C 217/08,** C07C 215/40, C07C 215/08, D06M 13/463, C11D 3/30, A61K 7/08

(21) Application number : **91200705.1**

(22) Date of filing : **27.03.91**

(54) **Alkoxy-(2-ethyl)hexyl-aliphatic methyl quaternary ammonium compounds and their precursor amines.**

(30) Priority : **04.04.90 US 505078**

(43) Date of publication of application : **09.10.91 Bulletin 91/41**

(45) Publication of the grant of the patent : **08.06.94 Bulletin 94/23**

(84) Designated Contracting States : **DE ES FR GB IT**

(56) References cited :
**EP-A- 0 090 117**
**EP-A- 0 098 802**
**DE-A- 3 116 087**
**US-A- 4 128 429**
**US-A- 4 199 465**
**US-A- 4 311 618**
**US-A- 4 569 800**

(73) Proprietor : **Akzo Nobel N.V.**
**Velperweg 76**
**NL-6824 BM Arnhem (NL)**

(72) Inventor : **Richmond, James Matthew**
**29 W. 304 Hartman Drive**
**Naperville, Illinois 60564 (US)**

(74) Representative : **Schalkwijk, Pieter Cornelis et al**
**AKZO NOBEL N.V.**
**Patent Department (Dept. CO)**
**P.O. Box 9300**
**NL-6800 SB Arnhem (NL)**

**EP 0 450 706 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a new class of amines and quaternary ammonium compounds. The amines and the quaternary ammonium compounds of the invention are ethoxylated and/or propoxylated (2-ethylhexyl) aliphatic compositions. The quaternary ammonium compounds of the current invention may be useful as fabric softeners and/or textile antistatic agents. They may also have use in hair-treating formulations as conditioners and/or antistatic agents.

US-A-4,569,800 discloses the compound

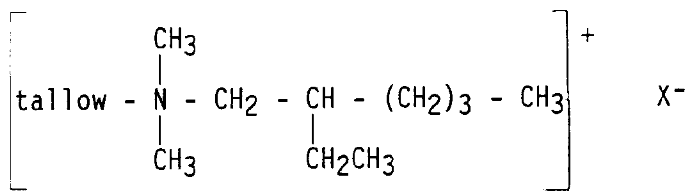

and its use in fabric softening environments.

Polyethoxylated and polypropoxylated quaternary ammonium salts are generally known. Methylbis(2-hydroxyethyl) tallowammonium chloride of the formula

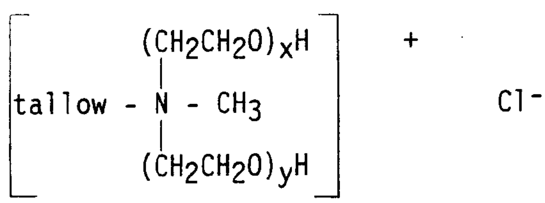

where x + y = 2 or 15 is known as a textile antistatic agent. Such quaternary compounds having 2 moles ethylene oxide are available under the tradename Ethoquad® T/12 from Akzo Chemicals Inc., 300 S. Riverside Plaza, Chicago, Illinois.

Although an excellent antistatic agent, methylbis(2-hydroxyethyl) tallowammonium chloride must be dissolved in a solvent, such as isopropyl alcohol, to be handleable. In the absence of a solvent, the quaternary ammonium chloride is a solid at room temperature and forms a gel with water.

JP-A-62255410 discloses the combined use of a 2-ethylhexyl quaternary ammonium compound and an ethoxylated phosphoric ester surfactant to provide antistatic properties and softness to hair.

Fabric conditioning compositions consisting of certain ethoxylated and/or propoxylated quaternary ammonium compounds, alcohol and hydrocarbon are known from US-A-4,139,477. US-A-3,625,891 discloses a fabric softener concentrate consisting essentially of a mixture of three quaternary ammonium compounds: a diaralkyl, a mixed - dialkyl and a dialkyl. Any of the three quaternary ammonium compounds may be ethoxylated.

There is, however, a continuing need in the art to provide quaternary ammonium compounds which may be easily blended into fabric softening formulations, fabric cleaning formulations and/or hair-treating formulations.

### The Invention

The invention, in one embodiment, is a quaternary ammonium compound of the general formula

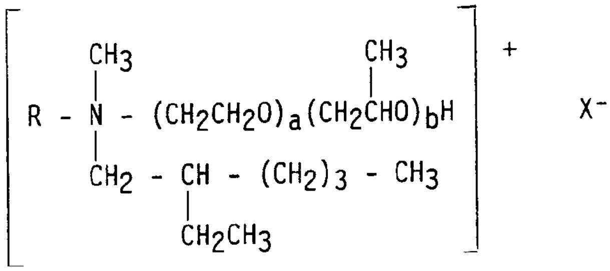

(I)

where R is an alkyl or alkenyl group having 8 to 22 carbon atoms, a is 0 to 50, b is 0 to 50, a + b ≧ 1 and X is an anion. The most preferred quaternary ammonium compounds of the current invention are (2-hydroxyethyl)-(2-ethylhexyl)-tallowalkylmethyl ammonium methylsulfate and (2-hydroxyethyl) polyoxyethyl(10)-(2-ethylhexyl) tallowalkylmethyl ammonium methylsulfate.

In another embodiment, the invention may be the tertiary amine of the following formula

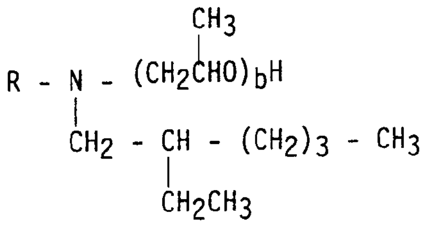

(II)

where R is an alkyl or alkenyl group having 8 to 22 carbon atoms, and b is 1 to 50. The invention is also drawn to salts of these amines, such as acetates, hydrochlorides and salts of fatty carboxylic acids.

It should be noted that tertiary amines satisfying the general formula

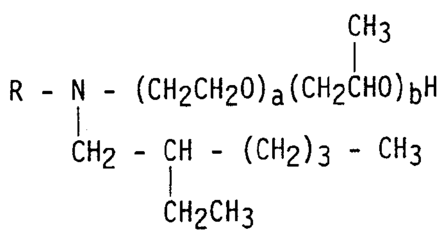

are disclosed in US-A-4,311,618. Specifically disclosed is the compound 2-(N-diisooctyl) aminoethyl pentaethyleneglycol ether, which corresponds to the above formula if R is an 2-ethylhexyl group, a is 6 and b is 0. Said compound finds use as such in a cleanser composition containing at least one nonionic surfactant with HLB value of 5-20, at least one amphoterically dissociating agent capable of breaking bridge bonds in cross-linked proteins, and at least one water-miscible or water-soluble aprotic lipophilic solvent. Not the slightest allusion is made to said amines being particularly useful as precursors to the quaternary ammonium compounds of formula (I) according to the present invention.

The compositions of the current invention have particular utility in fabric softening formulations, detergent formulations and hair-treating formulations.

Representative substituents of quaternary ammonium compounds (I) and tertiary amines (II) are discussed in more detail below.

R may be an organic group from the $C_8$ to $C_{22}$ alkyl series, such as octyl, nonyl, decyl, undecyl, dodecyl, tridecyl, tetradecyl, pentadecyl, hexadecyl, heptadecyl, octadecyl, nonyldecyl, eicoosanyl, heneicosanyl, docosanyl, ethyl hexyl, trimethyl hexyl, etc. Further, R may be an organic group of the type derived from natural sources, such as coco ($C_{12}$ and $C_{14}$), tallow ($C_{16}$ and $C_{18}$) and the like. R may also be an organic group from the alkenyl series, such as octenyl, nonenyl, decenyl, undecenyl, dodecenyl, tridecenyl, tetradecenyl, pentadecenyl, hexadecenyl, heptadecenyl, octadecenyl, nonyldecenyl, ethyl hexenyl, trimethyl hexenyl, etc.

In formula I, a and b may be, independently, any amount between 0 and about 50, with the caveat that either a or b is at least 1 (that is, a + b ≧ 1).

The anion X is preferably selected from the group chloride, bromide, methyl sulphate and ethyl sulphate.

The most preferred quaternary ammonium compounds of the current invention are (2-hydroxyethyl)-(2-ethylhexyl)-tallowalkylmethyl ammonium methylsulfate and (2-hydroxyethyl) polyoxyethyl(10)-(2-ethylhexyl)-tallowalkylmethyl ammonium methylsulfate.

The quaternary ammonium compounds of the current invention should find particular utility as fabric conditioning agents and fabric anti-static agents. Due to the melting point of these compounds, they are more likely useful with detergents in the wash cycle and in rinse cycle formulations than as in-dryer softeners.

Detergents. The art of formulating fabric detergent compositions is generally well-known. Such compositions vary greatly depending on the intended conditions of their use. The tables below provide non-limiting examples of possible liquid and powdered detergents comprising quaternary ammonium compounds of the current invention.

Table 1

Tradenames (or Commercial Names) and Sources of Detergent Formulation Constituents Used Herein

| Tradename (or Commercial Name)* | Chemical Description | Source |
|---|---|---|
| 1. Tergitol | Ethoxylated nonyl phenol | Union Carbide Corp. |
| 2. Ethanol SDA-40B | Alcoholic solvent | Generally available |
| 3. Ultrawet KX | Sodium linear alkylate sulfonate flake | ARCO Chemical |
| 4. Arctic White GT | Whitening agent | Hilton Davis Corp. |
| 5. Surfonic HDL | Blend of nonyl phenol (8.5 mole EO) and triethanol amine | Texaco Chem. Co. |
| 6. Neodol | Nonionic detergent | Shell Chemical Co. |
| 7. LAS-Na | Alkyl benzene sulfonate (average $C_{12}$ alkyl) | Generally available |
| 8. LAS-1/2 Mg | Alkyl benzene sulfonate (average $C_{12}$ alkyl) | Generally available |
| 9. AOS-1/2 Mg | Alpha olefin sulfonate | Generally available |
| 10. AOS-Na | Alpha olefin sulfonate | Generally available |
| 11. AES-1/2 Mg | Polyoxyethylene lauryl ether sulfate (typically 3 EO) | Generally available |
| 12. AES-Na | Polyoxyethylene lauryl ether sulfate (typically 3 EO) | Generally available |

* Na and 1/2 Mg indicate, respectively, neutralization with 1 mole NaOH or 1/2 mole $Mg(OH)_2$.

Table 2

Typical Liquid Detergent Formulations Comprised of A Quaternary Ammonium Compound of the Current Invention

| Component | Formulation A[1] (Wt.%) | Formulation B[2] (Wt.%) |
|---|---|---|
| Tergitol | 25.0 | 35.0 |
| Triethanolamine | 4.0 | 7.0 |
| Sulfuric Acid | 1.0 | 1.0 |
| Quaternary of This Invention | 5.3 | 8.0 |
| Ethanol SDA-40B | 10.0 | 11.0 |
| Water | 55.0 | 38.0 |

1 Useful for 1/2 cur per wash load.

2 Useful for 1/4 cup per wash load.

Table 3

Typical Liquid Detergent Formulation Comprised of A Quaternary Ammonium Compound of the Current Invention With Whiteness and Perfume

| Component | Formulation C (Wt.%) | Formulation D (Wt.%) |
|---|---|---|
| Sulfonic HDL | 21.5 | 21.5 |
| Ultrawet KX-90 | 3.0 | 3.0 |
| Quaternary of This Invention | 6.0 | 6.0 |
| Ethanol SD-40B | 8.0 | 8.0 |
| Arctic White GT | 0.3 | 0.3 |
| Water | 61.2 | 60.2 |
| Perfume | 0 | 1.0 |

Table 4

Typical Heavy Duty Liquid Detergent Formulations Comprised of A Quaternary Ammonium Compound of the Current Invention

| Component | Formulation E (Wt.%) | Formulation F (Wt.%) |
|---|---|---|
| Neodol | 27.0 | - |
| Triethanolamine | 4.5 | - |
| Surfonic HDL | - | 31.5 |
| Ultrawet KX-90 | 3.7 | 3.7 |
| Quaternary of This Invention | 7.5 | 7.5 |
| Ethanol SD-40B | 10.0 | 10.0 |
| Water | 47.3 | 47.3 |

Liquid detergent formulations comprising quaternaries of this invention may include many other components in addition to those provided in the tables herein. These components are well-known in the art. For example, brighteners, dyes and fragrances or perfumes may be added to the formulations. Typical brighteners include Interwite WGS (Crompton and Knowles Corp.) and Hiltamine Arctic White TX (Hilton Davis Corp.) Appropriate dyes are Nylanthrene Brilliant Blue GAW (Ciba-Geigy) and Orcoacid Alizarine Fast Blue SWA HI (Organic Chemical Corp.). Useful fragrances are Floral Fragrances 804609 and 817586 (Bush, Boake, Allen Inc.) and Detergent Softener Fragrance 286777 and 79219A (Haarmann and Reimer Corp.).

Table 5

Typical Powdered Detergent Formulations Comprised of A Quaternary Ammonium Compound of the Current Invention

| Component | G (Wt.%) | H | I | J | K | L | M |
|---|---|---|---|---|---|---|---|
| LAS-1/2 Mg | -- | 20 | 20 | -- | -- | -- | -- |
| LAS-Na | 20 | -- | -- | -- | -- | -- | -- |
| AOS-1/2 Mg | -- | -- | -- | 35 | -- | -- | -- |
| AOS-Na | -- | -- | -- | -- | 20 | -- | -- |
| AES-1/2 Mg | -- | -- | -- | -- | -- | 20 | -- |
| AES-Na | -- | -- | -- | -- | -- | -- | 30 |
| Na Tripolyphosphate | 20 | 20 | 20 | 20 | 20 | 20 | 20 |
| Na Carbonate | 15 | 15 | 15 | 15 | 15 | 15 | 15 |
| Quaternary of This Invention | 5 | 5 | 7,5 | 5 | 5 | 5 | 5 |
| Water | 10 | 10 | 10 | 10 | 10 | 10 | 10 |
| Na Sulfate | 30 | 30 | 27,5 | 15 | 30 | 30 | 20 |

Sodium tripolyphosphate and sodium carbonate are used as inorganic builders which improve detergency by removing calcium and magnesium ions. Sodium sulfate is used as a filler or bulking agent.

Preparation of granulated or powdered detergent is well-known in the art. For example, the formulation components may be mixed together, then prilled to form granules.

Fabric conditioning formulations. The quaternary compounds of this invention are also useful in fabric conditioning formulations added to the rinse cycle of a textile washing process to provide softness and/or anti-static characteristics to the textiles. Such formulations are typically aqueous dispersions of about 3-20 wt.% water-insoluble organic solids. It is, however, difficult to form and stabilize such dispersions. However, the high water dispersibility of the quaternary ammonium compounds of this invention should ease the preparation of such dispersions and increase their physical stability.

Hair-conditioners. The quaternary ammonium compounds of the current invention are also useful as components in after-shampoo hair conditioners in that the compounds are clear and they foster anti-static behavior. A typical hair conditioner would include about 0.5 to about 20 wt.% quaternary of this invention, the balance being water, perfume and dye. A detailed discussion of this art may be found in US-A-4,187,289.

Preparation of the tertiary amines and the quaternary ammonium compounds of this invention are more fully disclosed in the context of the following examples.

EXAMPLE 1

Preparation of Tertiary Amine: (2-hydroxyethyl)-(2-ethylhexyl)- tallowalkyl amine

To a 2-liter reaction vessel, 900 g (2.4 moles) tallow 2-ethylhexyl amine [$(C_{18}H_{37})NH(C_6H_{12})(C_2H_5)$] (available as Armeen® HTL8 secondary amine, a product of Akzo Chemicals Inc., 300 South Riverside Plaza, Chicago, Illinois), and 45 g of isopropyl alcohol were charged and heated to 120°C. Over a 4.5 hour period 114 g (2.6 moles) ethylene oxide were added to the reaction vessel. The mixture was digested for 2 (two) hours. The reaction vessel was vented at 125°C under nitrogen overpressure for 20 minutes. Analysis of the reaction product gave 97.6 wt.% (2.33 meg/g) (2-hydroxyethyl)-(2-ethylhexyl)-tallowalkyl amine and 1.2 wt.% (0.033

meq/g) residual primary and secondary amine. Further analysis of the reaction product found a color of 2 on the Gardner scale and a neutralization equivalent of (NE) of 423 from an HCl titration in isopropyl alcohol solvent (theoretical NE = 425).

EXAMPLE 2

Preparation of Quaternary Ammonium Methylsulfate: (2-hydroxyethyl)-(2-ethylhexyl)-tallowalkylmethyl ammonium methylsulfate

575 g (1.36 moles) tertiary amine from the above preparation were charged to a 2-liter reaction vessel. 171.3 g (1.36 moles) dimethyl sulfate (DMS) were added to the tertiary amine at 85°C over a 1 hour period and digested at 90°C for 0.5 hours. Analysis indicated 2.7% free amine. 14.5 g $Na_2CO_3$ were added and the mixture heated at 110°C for 1 hour producing 7.1% free amine. An additional 14 g DMS were added at 85°C and the mixture was digested at 90°C for 0.5 hours to produce 1.9% free amine. The reaction mixture temperature was reduced to 85°C and 40 g water and 7 g $Na_2CO_3$ were added. The mixture was stirred, the temperature was reduced to 70°C and held there for one hour. The final product was filtered. Analysis of the final product revealed 86.1 wt.% quaternary ammonium compound, 3.6 wt.% free amine, 6.2 wt.% water, .94 wt.% ash, Gardner color 3-4, pH 6.2, flash point >93°C (200°F), pour point -6°C (22°F) and a cloud point -11°C (13°F). The structure of (2-hydroxyethyl)-(2-ethylhexyl)-tallowalkylmethyl ammonium methylsulfate is

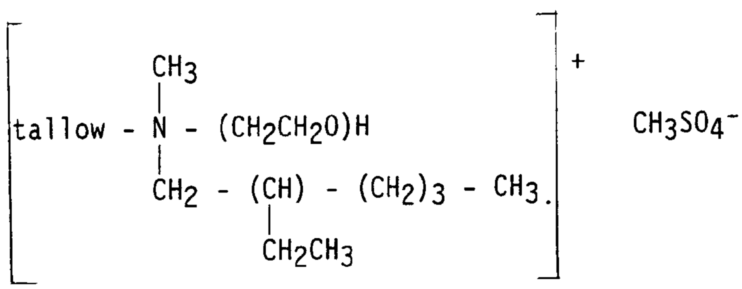

EXAMPLE 3

Preparation of Tertiary Amine: (2-hydroxyethyl)polyethoxy (10)-(2-ethylhexyl)-tallowalkyl amine

To a 2-liter reaction vessel, 423 g (1.0 mole) of the tertiary amine of Example 1, (2-hydroxyethyl)-(2-ethylhexyl)-tallowalkyl amine, 25 g of isopropyl alcohol, and 2 g of sodium acetate were charged and heated to 120°C. Over a five hour period 396 g (9.0 moles) of ethylene oxide were added to the reaction vessel. The mixture was digested for two hours, then vented at 125°C with a nitrogen overpressure. Analysis of the reaction product revealed that the title product was the principle product of the reaction.

EXAMPLE 4

Preparation of Quaternary Ammonium Methylsulfate: (2-hydroxyethyl)polyethoxy(10)-(2-ethylhexyl)-tallowalkylmethyl ammonium methylsulfate

A 2-liter reaction vessel was charged with 800 g (0.97 mole) of the tertiary amine of Example 3. 120 g (0.94 mole) of dimethyl sulfate (DMS) were added to the tertiary amine over a 1-hour period. The mixture was heated for an additional 30 minutes at 80°C. Analysis gave 96.3% quaternary, 1.2% amine salt and 3.1% free amine.

EXAMPLE A (COMPARATIVE)

Water Dispersibility of (2-Hydroxyethyl)-(2-ethylhexyl)-tallowalkylmethyl ammonium methylsulfate

Use of the compounds of the current invention typically requires dissolving, dispersing and/or emulsifying these compounds in water. This Example A demonstrates the superior water dispersibility of (2-hydroxyethyl)-(2-ethylhexyl)-tallowalkylmethyl ammonium methylsulfate, especially when compared to that of methylbis(2-hydroxyethyl)-tallowammonium chloride (Ethoquad® T/12 ammonium chloride, available from Akzo Chemicals

Inc., Chicago, Illinois).

The quaternary product from Example 2 above was diluted with water to a level of 50% water and 50% solids. The resulting product was a thin hazy liquid at room temperature that had a low viscosity approaching that of water. By contrast, methylbis(2-hydroxyethyl)-tallowalkyl ammonium chloride (Ethoquad® T/12 ammonium chloride), available from Akzo Chemicals Inc., Chicago, Illinois, was placed in a vessel and all solvents and water removed with the application of heat and vacuum. The resulting product was a white solid at room temperature. When this white solid was diluted with water to a level of 50% water and 50% solids, the resulting product was a thick syrup that had a gelatinous nature, which possessed a resistance to flow.

Ethoquad® T/12 ammonium chloride is a mixture of compounds having 16 or 18 carbon atoms in the hydrophobe and two hydrophilic ethylene oxide constituents. In contrast, the (2-hydroxyethyl)-(2-ethylhexyl)-tallowalkylmethyl ammonium methylsulfate of the instant invention has two hydrophobes; that is, a 2-ethylhexyl hydrophobe in addition to a 16 or 18 carbon atom hydrophobe. Further, (2-hydroxyethyl)-(2-ethylhexyl)-tallowalkylmethyl ammonium methylsulfate has only one hydrophilic ethylene oxide while Ethoquad T/12 ammonium chloride has two such hydrophilic groups. Such structural differences would suggest to a skilled artisan that Ethoquad® T/12 is more water dispersible than the compound of the instant invention. However, as shown by the data above, the opposite is surprisingly true.

## Claims

1. A quaternary ammonium compound of the general formula

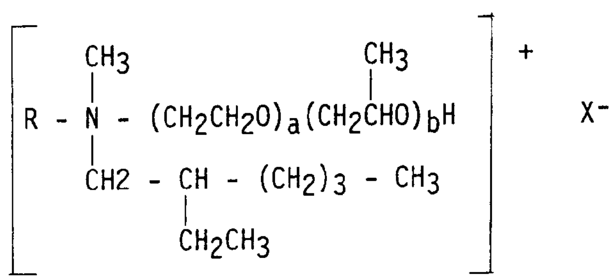

where R is an alkyl or alkenyl group having 8 to 22 carbon atoms, a is 0 to 50, b is 0 to 50, a + b ≧ 1 and X is an anion.

2. A quaternary ammonium compound of claim 1 wherein b is 0.

3. A quaternary ammonium compound of claim 1 wherein a is 0.

4. A quaternary ammonium compound of claim 1 wherein X is an anion selected from the group consisting of chloride, bromide, methylsulphate and ethylsulfate.

5. A quaternary ammonium compound of claim 1 wherein R is selected from the group consisting of (C12 and C14 alkyl) coco radicals and (C16 and C18 alkyl) tallow radicals.

6. A quaternary ammonium compound of claim 1 wherein R is (C16 and C18 alkyl) tallow, a is 1, b is 0 and X is methylsulphate.

7. A quaternary ammonium compound of claim 1 wherein R is (C16 and C18 alkyl) tallow, a is 11, b is 0 and X is methylsulphate.

8. A tertiary amine of the general formula

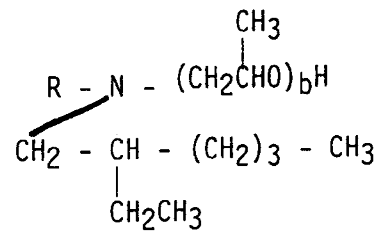

where R is an alkyl or alkenyl group having 8 to 22 carbon atoms and b is 1 to 50.

**9.** A tertiary amine of claim 8 wherein R is selected from the group consisting of (C12 and C14 alkyl) coco radicals and (C16 and C18 alkyl) tallow radicals.

**10.** A fabric softening formulation comprised of the quaternary ammonium compound of claim 1.

**11.** A detergent formulation comprised of the quaternary ammonium compound of claim 1.

**12.** A hair-treating formulation comprised of the quaternary ammonium compound of claim 1.

**Patentansprüche**

**1.** Quaternäre Ammoniumverbindung der allgemeinen Formel

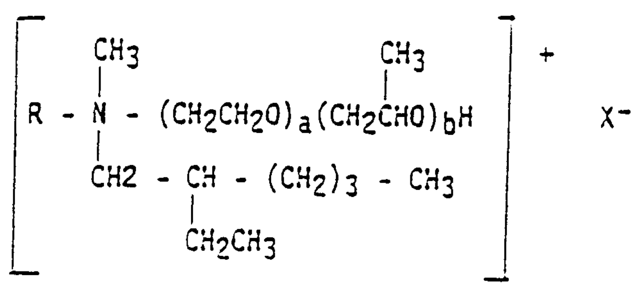

in der R eine Alkyl- oder Alkenylgruppe mit 8 bis 22 Kohlenstoffatomen, a = 0 bis 50, b = 0 bis 50, a + b ≧ 1 und X ein Anion ist.

**2.** Quaternäre Ammoniumverbindung nach Anspruch 1, in der b Null ist.

**3.** Quaternäre Ammoniumverbindung nach Anspruch 1, in der a Null ist.

**4.** Quaternäre Ammoniumverbindung nach Anspruch 1, in der X ein Anion aus der Gruppe Chlorid, Bromid, Methylsulfat und Ethylsulfat ist.

**5.** Quaternäre Ammoniumverbindung nach Anspruch 1, in der R gewählt ist aus ($C_{12}$- und $C_{14}$-Alkyl) Kokosalkylresten und ($C_{16}$- and $C_{18}$-Alkyl) Talgalkylresten.

**6.** Quaternäre Ammoniumverbindung nach Anspruch 1, in der R ein ($C_{16}$- und $C_{18}$-Alkyl) Talgalkylrest, a Eins, b Null und X Methylsulfat ist.

**7.** Quaternäre Ammoniumverbindung nach Anspruch 1, in der R ein ($C_{16}$- und $C_{18}$-Alkyl) Talgalkylrest, a = 11, b = Null und X Methylsulfat ist.

**8.** Tertiäres Amin der allgemeinen Formel

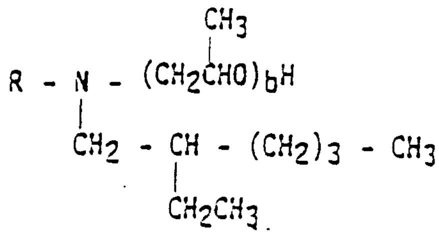

(II)

in der R eine Alkyl- oder Alkenylgruppe mit 8 bis 22 Kohlenstoffatomen und b 1 bis 50 ist.

9. Tertiäres Amin nach Anspruch 8, in dem R gewählt ist aus aus ($C_{12}$- und $C_{14}$-Alkyl) Kokosalkylresten und ($C_{16}$- und $C_{18}$-Alkyl) Talgalkylresten.

10. Gewebeweichmachermittel, das eine quaternäre Ammoniumverbindung nach Anspruch 1 enthält.

11. Waschmittel, das eine quaternäre Ammoniumverbindung nach Anspruch 1 enthält.

12. Haarbehandlungsmittel, das eine quaternäre Ammoniumverbindung nach Anspruch 1 enthält.

## Revendications

1. Composé d'ammonium quaternaire de formule générale :

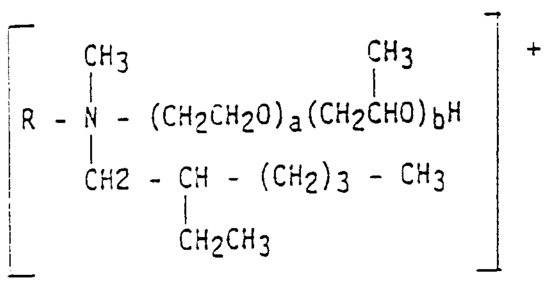

$X^-$

dans laquelle R représente un groupement alkyle ou alcényle en $C_8$-$C_{22}$, a est compris entre 0 et 50, b est compris entre 0 et 50, a + b ≧ 1 et X est un anion.

2. Composé d'ammonium quaternaire selon la revendication 1, dans lequel b vaut 0.

3. Composé d'ammonium quaternaire selon la revendication 1, dans lequel a vaut 0.

4. Composé d'ammonium quaternaire selon la revendication 1, dans lequel X est un anion choisi parmi les suivants : chlorure, bromure, méthylsulfate et éthylsulfate.

5. Composé d'ammonium quaternaire selon la revendication 1, dans lequel R est choisi parmi les radicaux de coco (groupements alkyle en $C_{12}$ et $C_{14}$) et les radicaux de suif (groupements alkyle en $C_{16}$ et $C_{18}$).

6. Composé d'ammonium quaternaire selon la revendication 1, dans lequel R représente un radical de suif (groupements alkyle en $C_{16}$ et $C_{18}$), a vaut 1, b vaut 0 et X est un anion méthylsulfate.

7. Composé d'ammonium quaternaire selon la revendication 1, dans lequel R représente un radical de suif (groupements alkyle en $C_{16}$ et $C_{18}$), a vaut 11, b vaut 0 et X est un anion méthylsulfate.

8. Amine tertiaire de formule générale

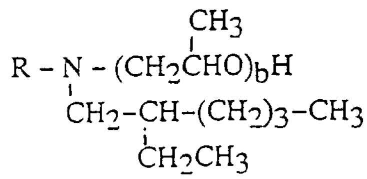

dans laquelle R représente un groupement alkyle ou alcényle en $C_8$-$C_{22}$ et b est compris entre 1 et 50.

**9.** Amine tertiaire selon la revendication 8, dans laquelle R est choisi dans le groupe constitué des radicaux de coco (groupements alkyle en $C_{12}$-$C_{14}$) et des radicaux de suif (groupements alkyle en $C_{16}$ et $C_{18}$).

**10.** Formulation d'adoucissant pour textile contenant le composé d'ammonium quaternaire de la revendication 1.

**11.** Formulation de détergent contenant le composé d'ammonium quaternaire de la revendication 1.

**12.** Formulation de traitement capillaire contenant le composé d'ammonium quaternaire de la revendication 1.